# EUROPEAN PATENT APPLICATION

(11) **EP 1 652 485 A1**
(43) Date of publication of application: **03.05.2006**
(21) Application number: 04025916.0
(22) Date of filing: 02.11.2004
(51) Int. Cl.: A61B 17/64, A61B 17/72

(54) **Tension guide fixator**

(71) Applicant: Rodrigues da Costa Martins, José Manuel, 2815-152 Ch. Caparica (PT)
(72) Inventor: Rodrigues da Costa Martins, José Manuel, 2815-152 Ch. Caparica (PT)

(57) **Abstract**

The present application refers to a External Fixator with Tension Guides device, called Tension Guide Fixator and it's constituted by several types of union rods (2) and (5), according with the surgery's need, articulated union cubes (6) and fixed or displaceable union cubes (7) used externally to the bone and intramedullar Tension Guides (8) used inside bone diaphisis, and it's a new stabilization treatment to long bone fractures, in any level, allowing the bone alignment, dynamis, compression and distraction.

## Description

The present utility model refers to a system that is constituted by union rods, external to the bone, connected to each other by fixed or displaceable union cubes and connected to the bone by threaded pins, and intramedullar tension guides that are connected to the external union rods by articulated union cubes, being used in the treatment of long bone's fractures, allowing not only the reduction of the fracture but also it's guidance in order to maintain the alignment and simultaneously the compression, dynamics and stretching of the fractured area, offering a new treatment option for the epiphisis fractures.

The originality of this model and it's working principle is based in a force system to which are applied the forces f₁ and f₂, directly to the tension guide A, obtaining the force f₃ by the formula f₁²+f₂²=f₃².

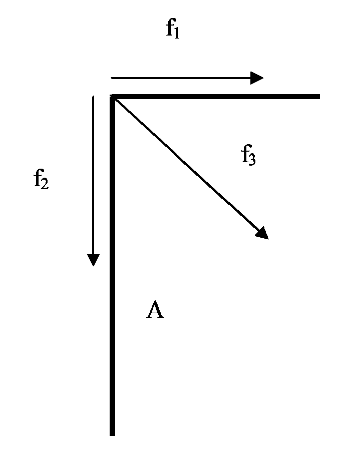

Until now there were only external fixators to long bones that were not directed to articular fractures. There is also no record of usage of internal tension guides that allow the alignment of the bone's fractured parts and the dynamics of the long bone.

The utility of the present model is easily explained by sketches:
- Picture 1 - the utility model, tension guide fixator, in one of it's positions.
- Picture 2 - simples union rods
- Picture 3 - threaded union rods
- Picture 4 - L-shaped union rods
- Picture 5 - double L-shaped union rods
- Picture 6 - articulated union cube
- Picture 7 - fixed or displaceable union cube
- Picture 8 - L-shaped intramedullar Tension Guide
- Picture 9 - intramedullar Tension Guide applicator

After the application of the intramedullar tension guide (picture 8), the threaded pins (Schanz pins - material available in the market) are putted into place in the bone diaphisis, connecting then to simple union rods (picture 2), or threaded rods (picture 3), to L-shaped (picture 4), or to double L-shaped (picture 5) by articulated union cubes (picture 6), according with the type of fracture and with the treatment objectives (dynamics, stabilization, compression or distraction). The union rods described above are connected to which other through fixed or displaceable union rods (picture 7). Finally the intramedullar tension guide (picture 8) is connected to the selected rod (picture 5) using an articulated union cube (picture 6).

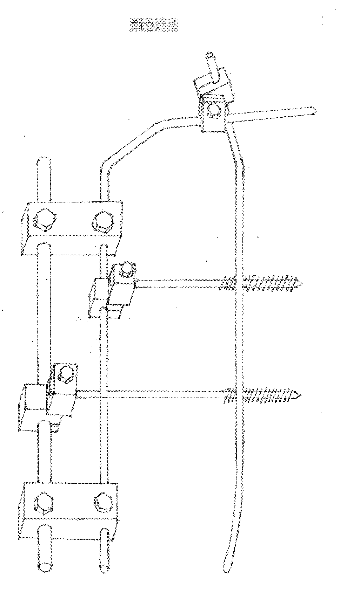

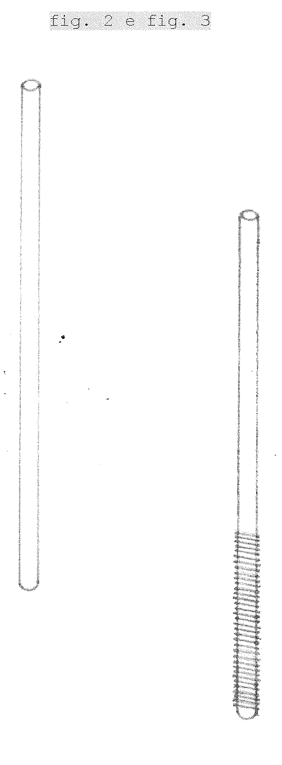

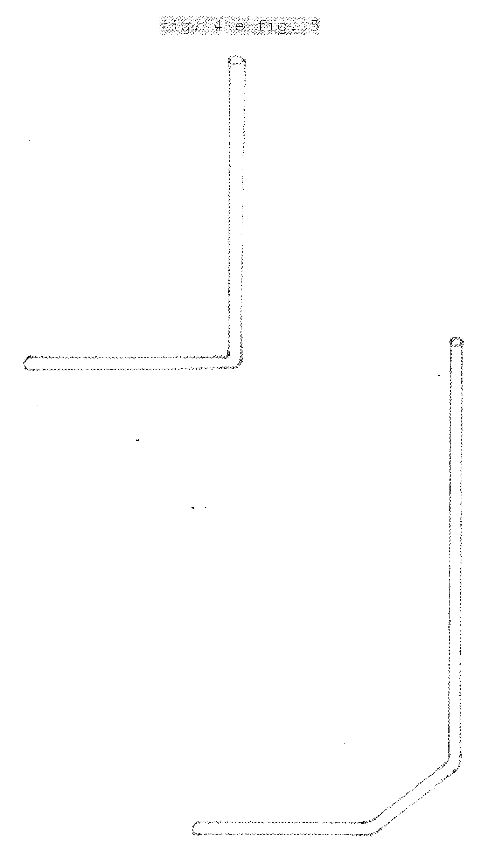

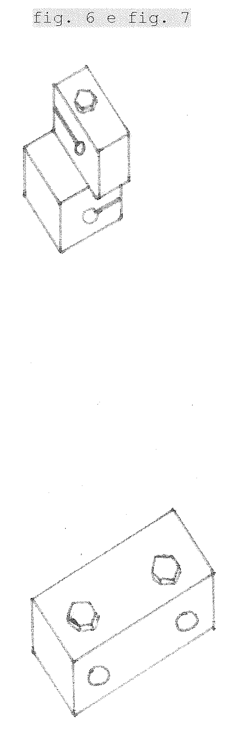

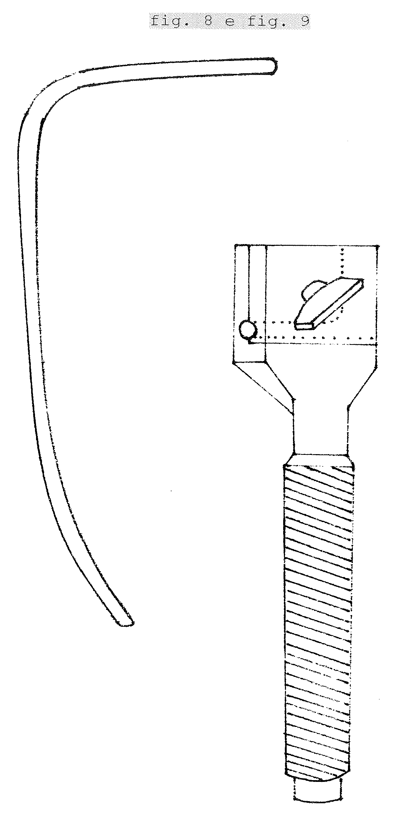

## Claims

1. This is a system created to the treatment of a lot of fractures in the long bones and epiphisis. The device, named "Tension Guide Fixator", is **characterized by** having several types of union rods (pictures 2,3,4 and 5), bounded to each other with fixed or displaceable union cubes (picture 7) and articulated with intramedullar tension guides by union cubes (picture 6), allowing the treatment of diaphisis and epiphisis fractures, particularly in the last case, to which it was specifically designed.

2. The device named "Intramedullar L-shaped Tension Guide" (picture 8), is **characterized by** it's union to the external portion of the external fixator, allowing the tension between the fracture fragments, keeping them connected and aligned, and at the same time guiding the bone development along it's axis.

3. Device according with position 2 and designed to apply the Intramedullar L-shaped Tension Guide (picture 9), **characterized by** it's specific shape that sustains the referred guide and allows it's intramedullar placement.
